# DEMANDE DE BREVET EUROPEEN

(11) **EP 1 721 897 A1**
(43) Date de publication de la demande: **15.11.2006**
(21) Numéro de dépôt: 06290758.9
(22) Date de dépôt: 11.05.2006
(51) Int. Cl.: C07D 213/72, A61K 31/44

(54) **Dérivés de phénylpyridinylpipérazine, leur procédé de préparation et les compositions pharmaceutiques qui les contiennent**

(30) Priorité: 12.05.2005 FR 0504758
(71) Demandeur: Les Laboratoires Servier, 92415 Courbevoie Cedex (FR)
(72) Inventeur: Desos, Patrice, 92270 Bois-Colombes (FR); Cordi, Alexis, 92150 Suresnes (FR); Lestage, Pierre, 78170 La Celle-St.-Cloud (FR)

(57) **Abrégé**

Composés de formule (I) : 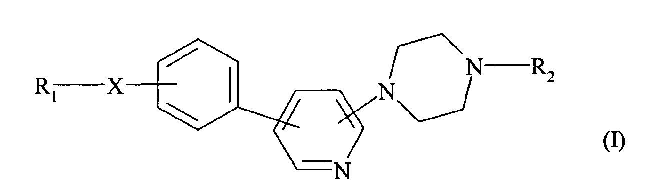 dans laquelle :
- X représente un groupement C(O) ou SO₂,
- R₁ représente un groupement aryle, ou un groupement NR₃R₄ dans lequel R₃ et R₄ sont tels que définis dans la description
- R₂ représente un groupement alkyle, cycloalkyle (C₃-C₈) ou cycloalkyle (C₃-C₈) alkyle (C₁-C₆),

leurs isomères ainsi que leurs sels d'addition.

Médicaments.

## Description

La présente invention concerne de nouveaux dérivés de phénylpyridinylpipérazine, leur procédé de préparation et les compositions pharmaceutiques qui les contiennent.

Les composés de la présente invention sont particulièrement intéressants d'un point de vue pharmacologique pour leur interaction spécifique avec les récepteurs histaminergiques centraux de type H₃, trouvant leur application dans le traitement des neuropathologies associées au vieillissement cérébral, des troubles de l'humeur, du comportement alimentaire et du rythme veille-sommeil, ainsi que du syndrome d'hyperactivité avec déficits attentionnels.

Le vieillissement de la population par augmentation de l'espérance de vie à la naissance a entraîné parallèlement un large accroissement de l'incidence des neuropathologies liées à l'âge et notamment de la maladie d'Alzheimer. Les principales manifestations cliniques du vieillissement cérébral et surtout des neuropathologies liées à l'âge, sont les déficits des fonctions mnésiques et cognitives qui peuvent conduire à la démence.

Au niveau du système nerveux central, de récentes études neuropharmacologiques ont montré que l'histamine *via* les systèmes histaminergiques centraux jouait un rôle de neurotransmetteur ou neuromodulateur en situations physiologiques ou physiopathologiques (Annu. Rev. Neurosci., 1986, 9, 209-254; Physiol. Rev., 1991, 71, 1-51). Ainsi, il a été montré que l'histamine intervenait dans divers processus physiologiques et comportementaux tels que la thermorégulaton, la régulation neuro-endocrinienne, le rythme circadien, les états cataleptiques, la motricité, l'agressivité, le comportement alimentaire, l'apprentissage et la mémorisation, ainsi que la plasticité synaptique (Hass et coll., histaminergic neurones : morphology and function, Boca Raton, FL : CRC Press, 1991, pp. 196-208 ; Prog. Neurobiology, 2001, 63, 637-672).

Parmi les 3 sous-types de récepteurs (H₁, H₂ et H₃) à l'histamine, il a été initialement montré que le récepteur de type H₃ était un autorécepteur pré-synaptique qui contrôlait la libération de l'histamine (Nature, 1987, 327, 117-123). Son activation inhibe la libération et la synthèse d'histamine par un mécanisme de feedback négatif (Neuroscience, 1987, 23, 149-157). Par la suite, il a été montré l'existence d'hétérorécepteurs pré-synaptiques, capables de moduler la libération de quelques neuropeptides et de nombreux neurotransmetteurs tels que noradrénaline, sérotonine, dopamine, GABA, acétylcholine et glutamate (TiPS, 1998, 19, 177-183). Des études, réalisées chez l'animal, ont montré que l'augmentation des taux endogènes extra-synaptiques d'histamine *via* le blocage des récepteurs de type H₃ par des antagonistes H₃ permettait de promouvoir les états de vigilance, les processus d'apprentissage et de mémoire, de réguler la prise alimentaire, et de s'opposer à des crises convulsives (Prog. Neurobiol., 2000, 63, 637-672; Neurosci. Biobehav. Rev., 2000, 24, 107-113). En conséquence, les indications thérapeutiques potentielles pour des antagonistes H₃ sont le traitement des déficits cognitifs associés au vieillissement cérébral et aux maladies neurodégénératives telles que la maladie d'Alzheimer, la maladie de Parkinson, la maladie de Pick, la maladie de Korsakoff et les démences frontales ou sous-corticales d'origine vasculaires ou autres, ainsi que le traitement des troubles de l'humeur, des crises convulsives, du syndrome d'hyperactivité avec déficits attentionnels, de l'obésité, de la douleur et des états narcoleptiques.

Les composés de la présente invention, outre leur originalité structurale, présentent des propriétés pharmacologiques tout à fait surprenantes et intéressantes dans ce domaine.

Plus spécifiquement, la présente invention concerne les composés de formule (I) : 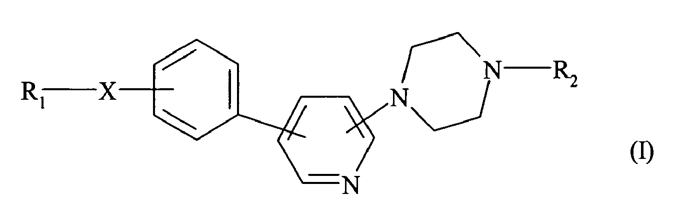 dans laquelle :
- X: représente un groupement C(O) ou SO₂,
- R₁: représente:
- un groupement aryle,
- ou un groupement NR₃R₄ dans lequel R₃ et R₄, identiques ou différents, représentent chacun un atome d'hydrogène ou un groupement alkyle (C₁-C₆) linéaire ou ramifié, cycloalkyle (C₃-C₈) ou cycloalkyl (C₃-C₈) alkyle (C₁-C₆) linéaire ou ramifié,
   ou R₃ et R₄ forment ensemble avec l'atome d'azote qui les porte un cycle comportant de 5 à 8 chaînons dont un des atomes de carbone peut être remplacé par un atome d'azote, d'oxygène ou de soufre ou par un groupement SO ou SO₂, le cycle ainsi défini pouvant être ponté par une chaîne alkyle (C₁-C₆) linéaire ou ramifiée, et/ou pouvant être éventuellement substitué par un ou plusieurs groupements, identiques ou différents, choisis parmi halogène, alkyle (C₁-C₆) linéaire ou ramifié, cycloalkyle (C₃-C₈), alkoxy (C₁-C₆) linéaire ou ramifié, polyhalogénoalkyle (C₁-C₆) linéaire ou ramifié, carboxy, hydroxy, cyano, oxo, nitro, amino (éventuellement substitué par un ou plusieurs groupements alkyle (C₁-C₆) linéaire ou ramifié),
- R₂: représente un groupement alkyle (C₁-C₆) linéaire ou ramifié, un groupement cycloalkyle (C₃-C₈), ou un groupement cycloalkyle (C₃-C₈) alkyle (C₁-C₆) dans lequel la partie alkyle peut être linéaire ou ramifiée,
leurs énantiomères et diastéréoisomères ainsi que leurs sels d'addition à un acide ou à une base pharmaceutiquement acceptable,
étant entendu que :
par groupement aryle, on comprend les groupements phényle, naphtyle et biphényle, ces groupements étant éventuellement substitués par un ou plusieurs groupements, identiques ou différents, choisis parmi halogène, alkyle (C₁-C₆) linéaire ou ramifié, alkoxy (C₁-C₆) linéaire ou ramifié, polyhalogénoalkyle (C₁-C₆) linéaire ou ramifié, carboxy, hydroxy, cyano, nitro, amino (éventuellement substitué par un ou plusieurs groupements alkyle (C₁-C₆) linéaire ou ramifié).

Parmi les acides pharmaceutiquement acceptables, on peut citer, à titre non limitatif, les acides chlorhydrique, bromhydrique, sulfurique, phosphonique, acétique, trifluoroacétique, lactique, pyruvique, malonique, succinique, glutarique, fumarique, tartrique, maléïque, citrique, ascorbique, méthane sulfonique, camphorique, etc...

Parmi les bases pharmaceutiquement acceptables, on peut citer, à titre non limitatif, l'hydroxyde de sodium, l'hydroxyde de potassium, la triéthylamine, la tertbutylamine, etc...

Les composés préférés selon l'invention sont les composés de formule (I) pour lesquels R₁ représente un groupement NR₃R₄.

Plus particulièrement, l'invention concerne les composés de formule (I) pour lesquels R₃ et R₄ forment ensemble avec l'atome d'azote qui les porte un cycle comportant de 5 à 8 chaînons dont un des atomes de carbone peut être remplacé par un atome d'azote, d'oxygène ou de soufre ou par un groupement SO ou SO₂, le cycle ainsi défini pouvant être ponté par une chaîne alkyle, et pouvant être non substitué ou substitué, préférentiellement par un ou plusieurs atomes d'halogène comme le fluor par exemple, ou par un groupement alkyle comme le groupement méthyle par exemple.

Encore plus préférentiellement, les groupements R₁ préférés sont les groupements morpholinyle, thiomorpholinyle, pipéridinyle, pipérazinyle, 4-(alkyl)pipérazinyle, pyrrolidinyle, 2-(alkyl)-2,5-diazabicyclo[2.2.1]heptanyle, 2-oxa-5-azabicyclo[2.2.1]heptanyle.

X représente avantageusement un groupement SO₂.

Le groupement R₂ préféré est le groupement cycloalkyle ou alkyle(C₂-C₆) et plus préférentiellement les groupements éthyle, isopropyle, cyclopentyle.

Encore plus particulièrement, l'invention concerne les composés de formule (I) qui sont :
- le 4-({4-[6-(4-isopropylpipérazin-1-yl)pyridin-3-yl]phényl}sulfonyl)morpholine dichlorhydrate,
- le 1-isopropyl-4-{5-[4-(pipéridin-1-ylsulfonyl)phényl]pyridin-2-yl}pipérazine dichlorhydrate,
- le 1-cyclopentyl-4-{5-[4-(pipéridin-1-ylsulfonyl)phényl]pyridin-2-yllpipérazine dichlorhydrate,
- le 1-cyclopropyl-4-{5-[4-(pipéridin-1-ylsulfonyl)phényl]pyridin-2-yl}pipérazine dichlorhydrate,
- le 1-éthyl-4-{5-[4-(pipéridin-1-ylsulfonyl)phényl]pyridin-2-yl}pipérazine dichlorhydrate,
- le 1-cyclobutyl-4-{5-[4-(pipéridin-1-ylsulfonyl)phényl]pyridin-2-yl}pipérazine dichlorhydrate,
- le 1-(5-{4-[(4,4-difluoropipéridin-1-yl)sulfonyl]phényl}pyridin-2-yl)-4-isopropyl pipérazine dichlorhydrate,
- le 4-({4-[6-(4-cyclopropylpipérazin-1-yl)pyridin-3-yl]phényl}sulfonyl)morpholine dichlorhydrate,
- le 1-isopropyl-4-(5-{4-[(4-méthylpipérazin-1-yl)sulfonyl]phényl}pyridin-2-yl) pipérazine trichlorhydrate,
- le 4-({4-[6-(4-isopropylpipérazin-1-yl)pyridin-3-yl]phényl}sulfonyl)thiomorpholine dichlorhydrate,
- le 1-cyclopentyl-4-{5-[4-(phénylsulfonyl)phényl]pyridin-2-yl}pipérazine dichlorhydrate,
- le 1-cyclopentyl-4-{5-[3-(pipéridin-1-ylsulfonyl)phényl]pyridin-2-yl}pipérazine dichlorhydrate,
- le 1-isopropyl-4-{5-[4-(pyrrolidin-1-ylsulfonyl)phényl]pyridin-2-yl}pipérazine dichlorhydrate,
- le 4-[6-(4-isopropylpipérazin-1-yl)pyridin-3-yl]-*N*,*N*-diméthylbenzènesulfonamide dichlorhydrate,
- le *N*-cyclopentyl-4-[6-(4-isopropyl-1-pipérazinyl)-3-pyridinyl]benzènesulfonamide,
- le 1-cyclopentyl-4-{5-[4-(pipéridin-1-ylcarbonyl)phényl]pyridin-2-yl}pipérazine dichlorhydrate,
- le 1-isopropyl-4-{5-[4-(pipéridin-1-ylcarbonyl)phényl]pyridin-2-yl}pipérazine dichlorhydrate,
- le 1-méthyl-4-{5-[4-(pipéridin-1-ylsulfonyl)phényl]pyridin-2-yl}pipérazine dichlorhydrate,
- le *N*-cyclopropyl-4-[6-(4-isopropylpipérazin-1-yl)pyridin-3-yl]benzène sulfonamide dichlorhydrate,
- *N*-(*tert*-butyl)-4-[6-(4-isopropylpipérazin-1-yl)pyridin-3-yl]benzène sulfonamide dichlorhydrate,
- 4-({4-[6-(4-isobutylpiperazin-1-yl)pyridin-3-yl]phenyl}sulfonyl)morpholine dichlorhydrate,
- 1-isopropyl-4-({4-[6-(4-isopropylpiperazin-1-yl)pyridin-3-yl]phenyl}sulfonyl) piperazine trichlorhydrate,
- 4-[6-(4-isopropylpipérazin-1-yl)pyridin-3-yl]benzènesulfonamide dichlorhydrate,
- 4-({4-[6-(4-isopropylpipérazin-1-yl)pyridin-3-yl]phényl}sulfonyl)thiomorpholine-1,1-dioxide dichlorhydrate,
- 1-éthyl-4-({4-[6-(4-isopropylpipérazin-1-yl)pyridin-3-yl]phényl}sulfonyl)pipérazine trichlorhydrate,
- 4-({4-[6-(4-isopropylpipérazin-1-yl)pyridin-3yl]phényl}sulfonyl)thiomorpholine-1-oxide dichlorhydrate,
- 1-{5-[4-(aziridin-1-ylsulfonyl)phényl]pyridin-2-yl}-4-isopropylpipérazine dichlorydrate,
- 1-isopropyl-4-(5-{4-[(2-méthylpyrrolidin-1-yl)sulfonyl]phényl}pyridin-2-yl)pipérazine dichlorhydrate,
- 1-isopropyl-4-{5-[4-(pipérazin-1-ylsulfonyl)phényl]pyridin-2-yl}pipérazine trichlorhydrate,
- 1-cyclohexyl-4-({4-[6-(4-isopropylpipérazin-1-yl)pyridin-3-yl]phényl}sulfonyl)pipérazine dichlorhydrate,
- 1-({4-[6-(4-isopropylpipérazin-1-yl)pyridin-3-yl]phényl}sulfonyl)pipéridin-4-one dichlorhydrate,
- 1-isopropyl-4-(5-{4-[(2-méthylpyrrolidin-1-yl)sulfonyl]phényl}pyridin-2-yl)pipérazine dichlorhydrate énantiomère 1,
- 1-isopropyl-4-(5-{4-[(2-méthylpyrrolidin-1-yl)sulfonyl]phényl}pyridin-2-yl)pipérazine dichlorhydrate énantiomère 2,
- 2-({4-[6-(4-isopropylpipérazin-1-yl)pyridin-3-yl]phényl}sulfonyl)-5-méthyl-2,5-diazabicyclo[2.2.1]heptane,
- 1-({4-[6-(4-isopropylpipérazin-1-yl)pyridin-3-yl]phényl}sulfonyl)-N,N-diméthylpipéridin-4-amine trichlorhydrate,
- 1-cyclopentyl-4-(5-{4-[(4-méthylpipérazin-1-yl)sulfonyl]phényl}pyridin-2-yl)pipérazine trichlorhydrate,
- 1-({4-[6-(4-isopropylpipérazin-1-yl)pyridin-3-yl]phényl}sulfonyl)pipéridin-4-ol dichlorhydrate,
- 1-isopropyl-4-(5-{3-[(4-méthylpipérazin-1-yl)sulfonyl]phényl}pyridin-2-yl)pipérazine trichlorhydrate,
- 1-(5-{4-[(4-fluoropipéridin-1-yl)sulfonyl]phényl}pyridin-2-yl)-4-isopropylpipérazine dichlorhydrate,
- 4-{4-[6-(4-isopropylpipérazin-1-yl)pyridin-3-yl]benzoyl}morpholine dichlorhydrate,
- 1-isopropyl-4-(5-{4-[(4-méthylpipérazin-1-yl)carbonyl]phényl}pyridin-2-yl)pipérazine trichlorhydrate,
- 1-({4-[6-(4-isopropylpipérazin-1-yl)pyridin-3-yl]phényl}sulfonyl)-*N*-méthylpipéridin-4-amine, trichlorhydrate,
- (1*S*,4*S*)-5-({4-[6-(4-isopropylpipérazin-1-yl)pyridin-3-yl]phényl}sulfonyl)-2-oxa-5-azabicyclo[2.2.1]heptane dichlorhydrate,
- 1-({4-[6-(4-isopropylpipérazin-1-yl)pyridin-3-yl]phényl}sulfonyl)pipéridin-4-amine trichlorhydrate.

L'invention concerne également le procédé de préparation des composés de formule (I) caractérisé en ce que l'on utilise comme produit de départ le composé de formule (II) : 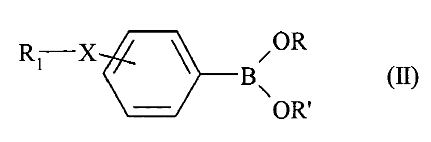 dans laquelle R₁ et X sont tels que définis dans la formule (I) et R et R', identiques ou différents, représentent chacun un atome d'hydrogène ou un groupement alkyle (C₁-C₆) linéaire ou ramifié, ou forment ensemble une chaîne alkylène (C₁-C₆) linéaire ou ramifiée, sur lequel on condense en présence de palladium (0) un composé de formule (III) : 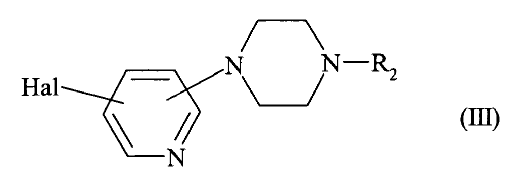 dans laquelle R₂ est tel que défini dans la formule (I) et Hal représente un atome d'halogène, pour conduire au composé de formule (I),
composé de formule (I) que l'on purifie, le cas échéant, selon une technique classique de purification, dont on sépare éventuellement les isomères, selon une technique classique de séparation, et que l'on transforme, si on le souhaite, en ses sels d'addition, à un acide ou à une base pharmaceutiquement acceptable.

Les composés de formule (II) et (III), tels que définis précédemment, sont commerciaux ou obtenus par des réactions classiques de la chimie organique.

Les composés de la présente invention, de par leurs propriétés pharmacologiques de ligands histaminiques du récepteur H₃, sont utiles dans le traitement des déficits cognitifs associés au vieillissement cérébral et aux maladies neurodégénératives telles que la maladie d'Alzheimer, la maladie de Parkinson, la maladie de Pick, la maladie de Korsakoff et les démences frontales ou sous-corticales d'origine vasculaires ou autres, ainsi que le traitement des troubles de l'humeur, des crises convulsives, du syndrome d'hyperactivité avec déficits attentionnels, de l'obésité, de la douleur et des états narcoleptiques.

La présente invention a également pour objet les compositions pharmaceutiques renfermant comme principe actif au moins un composé de formule (I), un de ses isomères, ou un de ses sels d'addition à un acide ou à une base pharmaceutiquement acceptable, seul ou en combinaison avec un ou plusieurs excipients ou véhicules inertes, non toxiques, pharmaceutiquement acceptables.
Parmi les compositions pharmaceutiques selon l'invention, il sera cité plus particulièrement celles qui conviennent pour l'administration orale, parentérale (intraveineuse, intramusculaire, ou sous-cutanée), per ou trans-cutanée, intravaginale, rectale, nasale, perlinguale, buccale, oculaire ou respiratoire.

Les compositions pharmaceutiques selon l'invention pour les injections parentérales comprennent notamment les solutions stériles aqueuses et non aqueuses, les dispersions, les suspensions ou émulsions ainsi que les poudres stériles pour la reconstitution des solutions ou des dispersions injectables.

Les compositions pharmaceutiques selon l'invention, pour les administrations orales solides, comprennent notamment les comprimés simples ou dragéifiés, les comprimés sublinguaux, les sachets, les gélules, les granules, et pour les administrations liquides orales, nasales, buccales ou oculaires, comprennent notamment les émulsions, les solutions, les suspensions, les gouttes, les sirops et les aérosols.

Les compositions pharmaceutiques pour l'administration rectale ou vaginale sont préférentiellement des suppositoires, et celles pour l'administration per ou trans-cutanée comprennent notamment les poudres, les aérosols, les crèmes, les pommades, les gels et les patchs.

Les compositions pharmaceutiques citées précédemment illustrent l'invention mais ne la limitent en aucune façon.

Parmi les excipients ou véhicules inertes, non toxiques, pharmaceutiquement acceptables, on peut citer à titre indicatif et non limitatif les diluants, les solvants, les conservateurs, les agents mouillants, les émulsifiants, les agents dispersants, les liants, les agents gonflants, les agents désintégrants, les retardants, les lubrifiants, les absorbants, les agents de suspension, les colorants, les aromatisants, etc...

La posologie utile varie selon l'âge et le poids du patient, la voie d'administration, la composition pharmaceutique utilisée, la nature et la sévérité de l'affection, et la prise de traitements associés éventuels. La posologie s'échelonne de 10 mg à 1 g en une ou plusieurs prises par jour.
Les préparations et exemples suivants illustrent l'invention mais ne la limitent en aucune façon.

Les produits de départ utilisés sont des produits connus ou préparés selon des modes opératoires connus.
Les structures des composés, décrits dans les exemple, ont été déterminées selon les techniques spectrophotométriques usuelles (infrarouge, RMN, spectrométrie de masse...).

### Préparation 1 : 1-(5-Bromopyridin-2-yl)-4-isopropylpipérazine

Une solution contenant 12,1 g de 2,5-dibromopyridine (51,1 mmol), 8,8 ml de 1-isopropylpipérazine (61,5 mmol) et 9,2 ml de DBU (61,5 mmol) est agitée une nuit à 100 °C. La réaction est ramenée à température ambiante et la solution diluée dans de l'eau et extraite par de l'acétate d'éthyle. Les phases organiques sont rassemblées, lavées par de la saumure, séchées (MgSO₄) et évaporées sous pression réduite. Le résidu est chromatographié sur colonne de SiO₂ en éluant par un mélange CH₂Cl₂/MeOH 98/2 puis 96/4 pour conduire au produit du titre.
*Point de fusion : 76-78 °C*
*Microanalyse élémentaire :*

| | *C* | *H* | *N* | *Br* |
|---|---|---|---|---|
| *% théorique* | *50,72* | *6,38* | *14,79* | *28,12* |
| *% expérimental* | *50,96* | *6,47* | *14,53* | *28,33* |

### Préparation 2 : 1-(5-Bromopyridin-2-yl)-4-cyclopentylpipérazine

Mode opératoire identique à celui de la Préparation 1 en remplaçant la 1-isopropylpipérazine par la 1-cyclopentylpipérazine.
*Point de fusion : 127-128 °C*

### Préparation 3 : 1-(5-Bromopyridin-2-yl)-4-cyctopropylpipérazine

Mode opératoire identique à celui de la Préparation 1 en remplaçant la 1-isopropylpipérazine par la 1-cyclopropylpipérazine.
*Point de fusion : 110-115 °C*

### Préparation 4 : 1-(5-Bromopyridin-2-yl)-4-éthytpipérazine

Mode opératoire identique à celui de la Préparation 1 en remplaçant la 1-isopropylpipérazine par la 1-éthylpipérazine.
*Point de fusion : 66 °C*
*Microanalyse élémentaire :*

| | *C* | *H* | *N* |
|---|---|---|---|
| *% théorique* | *48,90* | *5,97* | *15,55* |
| *% expérimental* | *48,98* | *6,19* | *15,07* |

### Préparation 5 : 1-(5-Bromopyridin-2-yl)-4-cyclobutylpipérazine

Mode opératoire identique à celui de la Préparation 1 en remplaçant la 1-isopropylpipérazine par la 1-cyclobutylpipérazine.
*Point de fusion : 98-102 °C*

### Préparation 6 : 1-(5-Bromo-2-pyridinyl)-4-méthylpipérazine

Mode opératoire identique à celui de la Préparation 1 en remplaçant la 1-isopropylpipérazine par la 4-méthylpipérazine.
*Point de fusion :* 71-73 *°C*

### Préparation 7 : 1-(5-Bromo-2-pyridinyl)-4-isobutylpipérazine

Mode opératoire identique à celui de la Préparation 1 en remplaçant la 1-isopropylpipérazine par la 1-isobutylpipérazine.
*Point de fusion : 80 °C*
*Microanalyse élémentaire :*

| | *C* | *H* | *N* | *Br* |
|---|---|---|---|---|
| *% théorique* | *52,36* | *6,76* | *14, 09* | *26,79* |
| *% expérimental* | *52,28* | *6,87* | *13,63* | *26,41* |

### Exemple 1 : 4-({4-[6-(4-Isopropylpipérazin-1-yl)pyridin-3-yl]phényl}sulfonyl) morpholine dichlorhydrate

### Stade A : 4-[(4-Iodophényl)sulfonyl]morpholine

A une solution de 50 g de chlorure de 4-iodobenzènesulfonyle (0,165 mol) dans 500 ml de CH₂Cl₂, sont ajoutés 46 ml de triéthylamine (0,33 mol) puis, goutte à goutte, 17 ml de morpholine (0,198 mol). La réaction étant exothermique, le ballon est placé dans un bain de glace jusqu'à retour à température ambiante. La réaction est agitée 1 heure à température ambiante. Après lavage du milieu réactionnel par environ 100 ml de HC1 1N puis 100 ml d'eau, la phase organique est séchée (MgSO₄) et évaporée sous pression réduite. Le résidu solide ainsi obtenu est repris en suspension dans un peu d'éther isopropylique pour conduire après filtration et séchage sous vide au produit du titre.
*Point de fusion : 141-144 °C*

### Stade B : Acide [4-(morpholin-4-ylsulfonyl)phényl]boronique

A une solution de 25 g du composé obtenu au Stade A (70,8 mmol) et 26 ml de borate de triisopropyle dans 400 ml de THF refroidis à - 60°C, sont additionnés goutte à goutte en 45 minutes et sous léger courant d'azote, 53 ml d'une solution 1,6 M de BuLi (84,9 mmol) dans l'hexane. La solution réactionnelle est ensuite agitée 1 heure 30 à - 60°C puis est ramenée à température ambiante en 2 heures. La réaction est traitée par environ 100 ml de HC1 1N et est extraite 3 fois par de l'acétate d'éthyle. Les phases organiques sont rassemblées, lavées par de la saumure, séchées (MgSO₄) et évaporées sous pression réduite. Le résidu obtenu est chromatographié sur une colonne de SiO₂ en éluant par CH₂Cl₂ puis par un mélange CH₂Cl₂/MeOH 98/2 puis 95/5. Après évaporation des fractions, le résidu est trituré dans l'éther éthylique pour conduire après filtration au produit du titre.
*Point de fusion : 104-110 °C*

### Stade C : 4-({4-[6-(4-Isopropylpipérazin-1-yl)pyridin-3-yl]phényl}sulfonyl) morpholine dichlorhydrate

Dans un tricol sont introduits 10,88 g du composé obtenu dans la Préparation 1 (38,3 mmol), 13,5 g du composé obtenu au Stade B (49,8 mmol), 250 ml de dioxanne et 190 ml de Na₂CO₃ 0,4 M dans l'eau. Le milieu réactionnel est dégazé en faisant buller de l'azote pendant 30 min. Le Pd(0)tétrakistriphénylphosphine (2,21 g, 1,91 mmol) est introduit et la réaction est agitée à 100°C sous léger courant d'azote pendant 3 heures. Après refroidissement à température ambiante, le milieu réactionnel est dilué dans l'eau et extrait par de l'acétate d'éthyle. Au cours de l'extraction, il se forme un précipité qui est filtré, rincé par de l'eau et un peu d'acétate d'éthyle pour donner après séchage sous vide un premier lot du produit du titre sous forme de base. Les phases d'extractions sont jointes au filtrat et la phase organique est décantée puis lavée par de la saumure. La phase organique est séchée (MgSO₄) et évaporées sous pression réduite. Le résidu d'évaporation est repris en suspension dans de l'éthanol et filtré pour donner après séchage sous vide un deuxième lot du composé du titre sous forme de base. Les 2 lots sont joints et mis en suspension dans de l'éthanol. De l'éther chlorhydrique est ajouté et la suspension est filtrée pour conduire au produit du titre.
*Point de fusion : 254-256 °C*
*Microanalyse élémentaire :*

| | *C* | *H* | *N* | *S* | *Cl* |
|---|---|---|---|---|---|
| *% théorique* | *52,48* | *6,41* | *11,13* | *6,37* | *14,08* |
| *% expérimental* | *52,62* | *6,40* | *10,93* | *6,50* | *14,45* |

### Exemple 2 : 1-Isopropyl-4-{5-[4-(pipéridin-1-ylsulfonyl)phényl]pyridin-2-yl} pipérazine dichlorhydrate

### Stade A : 1-[(4-Iodophényl)sulfonyl]pipéridine

Mode opératoire identique au Stade A de l'exemple 1 en remplaçant la morpholine par la pipéridine.
*Microanalyse élémentaire :*

| | C | *H* | *N* | *S* | *I* |
|---|---|---|---|---|---|
| *% théorique* | *37, 62* | *4, 02* | *3,99* | *9,13* | *36,13* |
| *% expérimental* | *37,91* | *4,08* | *4,01* | *8,98* | *36,54* |

### Stade B : Acide [4-(pipéridin-1-ylsulfonyl)phényl]boronique

Mode opératoire identique au stade B de l'Exemple 1 à partir du produit obtenu au Stade A.
*Point de fusion :* 110 *°C*

### Stade C : 1-Isopropyl-4-{5-[4-(pipéridin-1-ylsulfonyl)phényl]pyridin-2-yl} pipérazine dichlorhydrate

Mode opératoire identique au Stade C de l'Exemple 1 à partir du produit obtenu au Stade B.
*Point de fusion : 249-252 °C*
*Microanalyse élémentaire :*

| | *C* | *H* | *N* | *S* | *Cl* |
|---|---|---|---|---|---|
| *% théorique* | *55,08* | *6,83* | *11,17* | *6,39* | *14,14* |
| *% expérimental* | *54,83* | *7, 00* | *11,05* | *6,18* | *13,74* |

### Exemple 3 : 1-Cyclopentyl-4-{5-[4-(pipéridin-1-ylsulfonyl)phényl]pyridin-2-yl} pipérazine dichlorhydrate

Mode opératoire identique au Stade C de l'Exemple 2 mais en remplaçant le produit de la Préparation 1 par le produit obtenu dans la Préparation 2.
*Point de fusion : 241-243 °C*
*Microanalyse élémentaire :*

| | *C* | *H* | *N* | *S* | *Cl* |
|---|---|---|---|---|---|
| *% théorique* | *56, 92* | *6,88* | *10, 62* | *6,08* | *13,44* |
| *% expérimental* | *56, 65* | *7,11* | *10,37* | *6,13* | *13, 07* |

### Exemple 4 : 1-Cyclopropyl-4-{5-[4-(pipéridin-1-ylsulfanyl)phényl]pyridin-2-yl} pipérazine dichlorhydrate

Le produit du Stade B de l'exemple 2 est mis en réaction avec le composé obtenu dans la Préparation 3 selon les conditions décrites dans le Stade C de l'Exemple 1.
*Point de fusion : 204-208 °C*
*Microanalyse élémentaire :*

| | *C* | *H* | *N* | *S* | *Cl* |
|---|---|---|---|---|---|
| *% théorique* | *55,31* | *6,46* | *11,22* | *6,42* | *14,20* |
| *% expérimental* | *56, 01* | *6,74* | *11,14* | *5,81* | *13,99* |

### Exemple 5 : 1-Ethyl-4-{5-[4-(pipéridin-1-ylsulfonyl)phényl]pyridin-2-yl}pipérazine dichlorhydrate

Le produit obtenu dans le Stade B de l'Exemple 2 est mis en réaction avec le composé obtenu dans la Préparation 4 selon les conditions décrites au stade C de l'Exemple 1.
*Point de fusion : 245-247 °C*
*Microanalyse élémentaire :*

| | *C* | *H* | *N* | *S* | *Cl* |
|---|---|---|---|---|---|
| *% théorique* | *54,20* | *6,62* | *11,49* | *6,58* | *14,54* |
| *% expérimental* | *54,87* | *7,01* | *11,56* | *6,12* | *14,92* |

### Exemple 6 : 1-Cyclobutyl-4-{5-[4-(pipéridin-1-ylsulfonyl)phényl]pyridin-2-yl}pipérazine dichlorhydrate

Le produit obtenu au Stade B de l'Exemple 2 est mis en réaction avec le composé obtenu dans la Préparation 5 selon les conditions décrites au Stade C de l'Exemple 1.
*Point de fusion : 250-253 °C*
*Microanalyse élémentaire :*

| | *C* | *H* | *N* | *S* | *Cl* |
|---|---|---|---|---|---|
| *% théorique* | *57,77* | *6,79* | *11,23* | *6,43* | *13,81* |
| *% expérimental* | *57,54* | *6,84* | *11,00* | *6, 04* | *11, 69* |

### Exemple 7 : 1-(5-{4-[(4,4-Difluoropipéridin-1-yl)sulfonyl]phényl}pyridin-2-yl)-4-isopropylpipérazine dichlorhydrate

### Stade A : 4,4-Difluoro-1-[(4-iodophényl)sulfonyl]pipéridine

Mode opératoire identique au Stade A de l'Exemple 1 en remplaçant la morpholine par la 4,4-difluoropipéridine.
*Point de fusion : 148-150 °C*
*Microanalyse élémentaire :*

| | *C* | *H* | *N* | *S* | *I* |
|---|---|---|---|---|---|
| *% théorique* | *34,12* | *3,12* | *3, 62* | *8, 28* | *32,78* |
| *% expérimental* | *34, 62* | *3, 27* | *3, 66* | *8,30* | *33,36* |

### Stade B : Acide {4-[(4,4-difluoropipéridin-1-yl)sulfonyl]phényl}boronique

Mode opératoire identique au Stade B de l'Exemple 1 à partir du produit obtenu au Stade A.
*Point de fusion : 289 °C*

### Stade C : 1-(5-{4-[(4,4-Difluoropipéridin-1-yl)sulfonyl]phényl}pyridin-2-yl)-4-isopropylpipérazine dichlorhydrate

Mode opératoire identique au stade C de l'Exemple 1 en utilisant le produit obtenu au stade B.
*Point de fusion : 260-262 °C*
*Microanalyse élémentaire :*

| | *C* | *H* | *N* | *S* | *Cl* |
|---|---|---|---|---|---|
| *% théorique* | *51,4* | *6,00* | *10,42* | *5,97* | *13,19* |
| *% expérimental* | *51, 07* | *5,85* | *10, 03* | *6, 07* | *13,84* |

### Exemple 8 : 4-({4-[6-(4-Cyclopropylpipérazin-1-yl)pyridin-3-yl]phényl}sulfonyl) morpholine dichlorhydrate

Le produit du Stade B de l'Exemple 1 est mis en réaction avec le composé obtenu dans la Préparation 3 selon les conditions décrites au Stade C de l'Exemple 1.
*Point de fusion : 220 °C*
*Microanalyse élémentaire :*

| | *C* | *H* | *N* | *S* | *Cl* |
|---|---|---|---|---|---|
| *% théorique* | *52, 69* | *6, 03* | *11,17* | *6,39* | *14,14* |
| *% expérimental* | *52, 67* | *6, 04* | *10,83* | *6,35* | *14, 22* |

### Exemple 9 : 1-Isopropyl-4-(5-{4-[(4-méthylpipérazin-1-yl)sulfonyl]phényl}pyridin-2-yl)pipérazine trichlorhydrate

### Stade A : 1-[(4-Iodophényl)sulfonyl]-4-méthylpipérazine

Mode opératoire identique au Stade A de l'Exemple 1 en remplaçant la morpholine par la 4-méthylpipérazine.
*Point de fusion : 181-182 °C*

### Stade B : 1-Méthyl-4-{[4-(4,4,5,5-tétraméthyl-1,3,2-dioxaborolan-2-yl)phényl]sulfonyl}pipérazine

Dans un bicol de 100 ml sont introduits 5,4 g du composé obtenu au Stade A (14,75 mmol), 4,52 g de bis-pinacolatodiborane (19,18 mmol), 4,34 g d'acétate de potassium (44,25 mmol) et 50 ml de diméthylformamide. Le milieu réactionnel est dégazé en barbotant un courant d'azote pendant 30 min, puis 165 mg d'acétate de palladium (0,737 mmol) sont ajoutés. La réaction est agitée sous léger courant d'azote pendant 2 heures 30 à 85°C. Après refroidissement à température ambiante, le milieu réactionnel est dilué dans de l'eau et extrait par CH₂Cl₂ . Les phases organiques sont collectées, lavées par de la saumure, séchées, évaporées sous pression réduite. Le résidu obtenu est chromatographié sur SiO₂ (CH₂Cl₂/ MeOH 95/5) pour conduire au produit du titre sous la forme d'un solide blanc crème.
*Point de fusion : 126-136 °C*

### Stade C : 1-Isopropyl-4-(5-{4-[(4-méthylpipérazin-1-yl)sulfonyl]phényl} pyridin-2-yl)pipérazine trichlorhydrate

Mode opératoire identique au Stade C de l'Exemple 1 à partir du produit obtenu au stade B précédent.
*Point de fusion : 254-258 °C*
*Microanalyse élémentaire :*

| | *C* | *H* | *N* | *S* | *Cl* |
|---|---|---|---|---|---|
| *% théorique* | *49,96* | *6,56* | *12, 66* | *5,80* | *19, 23* |
| *% expérimental* | *50,57* | *6,55* | *12,50* | *5,82* | *18,50* |

### Exemple 10 : 4-({4-[6-(4-Isopropylpipérazin-1-yl)pyridin-3-yl]phényl}sulfonyl) thiomorpholine dichlorhydrate

### Stade A : 4-[(4-Iodophényl)sulfonyl]thiomorpholine

Mode opératoire identique au Stade A de l'Exemple 1 en remplaçant la morpholine par la thiomorpholine.
*Point de fusion : 131 °C*

### Stade B : Acide [4-(thiomorpholin-4-ylsulfonyl)phényl]boronique

Mode opératoire identique au Stade B de l'Exemple 1 à partir du produit obtenu au Stade A.
*Point de fusion : >300 °C*

### Stade C : 4-({4-[6-(4-Isopropylpipérazin-1-yl)pyridin-3-yl]phényl}sulfonyl) thiomorpholine dichlorhydrate

Mode opératoire identique au Stade C de l'Exemple 1 à partir du produit obtenu dans le Stade B.
*Point de fusion : 248-253 °C*
*Microanalyse élémentaire :*

| | *C* | *H* | *N* | *S* | *Cl* |
|---|---|---|---|---|---|
| *% théorique* | *50,86* | *6, 21* | *10,78* | *12,34* | *13, 65* |
| *% expérimental* | *51,51* | *6,41* | *10,35* | *11,74* | *13,95* |

### Exemple 11 : 1-Cyclopentyl-4-{5-[4-(phénylsulfonyl)phényl]pyridin-2-yl}pipérazine dichlorhydrate

### Stade A : 1-Bromo-4-(phénylsulfonyl)benzène

A une solution de 199 µl de bromobenzène (1,88 mmol) et 361 µl de chlorure de benzène sulfonyle (2,83 mmol) dans 4 ml d'acide trifluoroacétique sont ajoutés successivement 83 mg de chlorure d'indium (0,376 mmol) puis goutte à goutte 25µl d'acide trifluorométhanesulfonique. La réaction est agitée 2 heures à 70°C puis ramenée à température ambiante et diluée dans de l'eau glacée. Après alcalinisation à pH 10 par addition de soude concentrée, le milieu réactionnel est extrait par CH₂Cl₂. Les phases organiques sont rassemblées, lavées par NaCl saturé, séchées (MgSO₄) et évaporées sous pression réduite pour conduire au produit du titre sous la forme d'un solide blanc.
*Point de fusion : 95-99 °C*
*Microanalyse élémentaire :*

| | *C* | *H* | *S* | *Br* |
|---|---|---|---|---|
| *% théorique* | *48,50* | *3,05* | *10,79* | *26,89* |
| *% expérimental* | *48,21* | *3,21* | *11,17* | *27,32* |

### Stade B : Acide [4-(phénylsulfonyl)phényl]boronique

Mode opératoire identique au Stade B de l'Exemple 1 à partir du produit obtenu au Stade A.
*Point de fusion : 287-290 °C*

### Stade C : 1-Cyclopentyl-4-{5-[4-(phénylsulfonyl)phényl]pyridin-2-yl} pipérazine dichlorhydrate

Mode opératoire identique au Stade C de l'Exemple 1 à partir du produit obtenu au Stade B et en remplaçant le produit de la Préparation 1 par le produit de la Préparation 2.
*Point de fusion : 155-159 °C*
*Microanalyse élémentaire :*

| | *C* | *H* | *N* | *S* | *Cl* |
|---|---|---|---|---|---|
| *% théorique* | *59,99* | *6, 00* | *8, 07* | *6,16* | *13,62* |
| *% expérimental* | *60,36* | *5,86* | *7,95* | *5,99* | *13,99* |

### Exemple 12 : 1-Cyclopentyl-4-{5-[3-(pipéridin-1-ylsulfonyl)phényl]pyridin-2-yl} pipérazine dichlorhydrate

### Stade A : 1-[(3-Bromophényl)sulfonyl]pipéridine

Mode opératoire identique au Stade A de l'Exemple 1 à partir du chlorure de 3-bromobenzènesulfonyle et de la pipéridine.
*Point de fusion : 87 °C*
*Microanalyse élémentaire :*

| | *C* | *H* | *N* | *S* | *Br* |
|---|---|---|---|---|---|
| *% théorique* | *43,43* | *4, 64* | *4, 60* | *10,54* | *26,27* |
| *% expérimental* | *43,71* | *4,75* | *4,72* | *11,02* | *26,37* |

### Stade B : Acide [3-(pipéridin-1-ylsulfonyl)phényl]boronique

Mode opératoire identique au Stade B de l'Exemple 1 à partir du produit obtenu au stade A.
*Point de fusion : 115-119 °C*

### Stade C : 1-Cyclopentyl-4-{5-[3-(pipéridin-1-ylsulfonyl)phényl]pyridin-2-yl} pipérazine dichlorhydrate

Mode opératoire identique au Stade C de l'Exemple 1 à partir du produit obtenu au Stade B et en remplaçant le composé de la Préparation 1 par le produit obtenu dans la Préparation 2.
*Point de fusion : 229-231 °C*
*Microanalyse élémentaire :*

| | *C* | *H* | *N* | *S* | *Cl* |
|---|---|---|---|---|---|
| *% théorique* | *56,92* | *6,88* | *10,62* | *6, 08* | *13,44* |
| *% expérimental* | *56,76* | *6,92* | *10,42* | *5,91* | *13,47* |

### Exemple 13 : 1-Isopropyl-4-{5-[4-(pyrrolidin-1-ylsulfonyl)phényl]pyridin-2-yl} pipérazine dichlorhydrate

### Stade A : 1-[(4-Iodophényl)sulfonyl]pyrrolidine

Mode opératoire identique au Stade A de l'Exemple 1 en remplaçant la morpholine par la pyrrolidine.
*Point de fusion : 126* °C
*Microanalyse élémentaire :*

| | *C* | *H* | *N* | *S* | *I* |
|---|---|---|---|---|---|
| *% théorique* | *35,62* | *3,59* | *4,15* | *9, 51* | *37,64* |
| *% expérimental* | *37,13* | *3,80* | *4,19* | *9, 29* | *36,89* |

### Stade B : Acide [4-(pyrrolidin-1-ylsulfonyl)phényl]boronique

Mode opératoire identique au Stade B de l'Exemple 1 à partir du produit obtenu au Stade A.
*Point de fusion : 306 °C*

### Stade C : 1-Isopropyl-4-{5-[4-(pyrrolidin-1-ylsulfonyl)phényl]pyridin-2-yl}pipérazine dichlorhydrate

Mode opératoire identique au Stade C de l'Exemple 1 à partir du produit obtenu au Stade B.
*Point de fusion : 240 °C*
*Microanalyse élémentaire :*

| | *C* | *H* | *N* | *S* | *Cl* |
|---|---|---|---|---|---|
| *% théorique* | *54,20* | *6, 62* | *11,49* | *6,58* | *14,54* |
| *% expérimental* | *54,32* | *6,54* | *11,18* | *6,57* | *15,23* |

### Exemple 14 : 4-[6-(4-Isopropylpipérazin-1-yl)pyridin-3-yl]-N,N-diméthylbenzène sulfonamide dichlorhydrate

### Stade A : 4-Iodo-N,N-diméthylbenzènesulfonamide

Mode opératoire identique au Stade A de l'Exemple 1 en remplaçant la morpholine par la diméthylamine.
*Point de fusion : 128 °C*
*Microanalyse élémentaire :*

| | *C* | *H* | *N* | *S* | *I* |
|---|---|---|---|---|---|
| *% théorique* | *30,88* | *3, 24* | *4,50* | *10,31* | *40,79* |
| *% expérimental* | *31,56* | *3,32* | *4,41* | *10,10* | *39,50* |

### Stade B : Acide {4-[(diméthylamino)sulfonyl]phényl}boronique

Mode opératoire identique au Stade B de l'Exemple 1 à partir du produit obtenu au stade A.
*Point de fusion : 306 °C*

### Stade C : 4-[6-(4-Isopropylpipérazin-1-yl)pyridin-3-yl]-N,N-diméthylbenzène sulfonamide dichlorhydrate

Mode opératoire identique au Stade C de l'Exemple 1 à partir du composé obtenu au stade B.
*Point de fusion : 240 °C*
*Microanalyse élémentaire :*

| | *C* | *H* | *N* | *S* | *Cl* |
|---|---|---|---|---|---|
| *% théorique* | *52,06* | *6,55* | *12,14* | *6,95* | *15,52* |
| *% expérimental* | *52,39* | *6,68* | *11,69* | *6,91* | *15,74* |

### Exemple 15 : N-Cyclopentyl-4-[6-(4-isopropyl-1-pipérazinyl)-3-pyridinyl]benzène sulfonamide dichlorhydrate

### Stade A : N-Cyclopentyl-4-iodobenzènesulfonamide

Mode opératoire identique au Stade A de l'Exemple 1 en remplaçant la morpholine par la cyclopentylamine.

### Stade B : N-Cyclopentyl-4-(4,4,5,5-tétraméthyl-1,3,2-dioxaborolan-2-yl)benzènesulfonamide

Mode opératoire identique au Stade B de l'Exemple 9 à partir du produit obtenu au stade A.

### Stade C : N-Cyclopentyl-4-[6-(4-isopropyl-1-pipérazinyl)-3-pyridinyl] benzènesulfonamide dichlorhydrate

Mode opératoire identique au Stade C de l'Exemple 1 à partir du composé obtenu au stade B.

### Exemple 16 : 1-Cyclopentyl-4-{5-[4-(pipéridin-1-ylcarbonyl)phényl]pyridin-2-yl}pipérazine dichlorhydrate

### Stade A : 1-(4-Iodobenzoyl)pipéridine

A une suspension de 4,0 g d'acide 4-iodobenzoïque (16,13 mmol) dans 40 ml de CH₂Cl₂ sont ajoutés 3,65 ml de diisopropyléthylamine (20,97 mmol) puis, après 10 min, 5,18 g de TBTU (16,13 mmol). Après 10 min supplémentaires d'agitation, 1,60 ml de pipéridine (16,13 mmol) sont ajoutés et la réaction est agitée une nuit à température ambiante. Le milieu réactionnel est lavé 3 fois par de l'eau puis 1 fois par NaCl saturé. Après séchage (MgSO₄) et évaporation sous pression réduite le résidu est chromatographié sur silice (CH₂Cl₂/acétone 9/1) pour conduire au produit du titre.
*Point de fusion : 115-118 °C*

### Stade B : Acide [4-(pipéridin-1-ylcarbonyl)phényl]boronique

Mode opératoire identique au Stade B de l'Exemple 1 à partir du composé obtenu au stade A.
*Point de fusion : 135-140 °C*

### Stade C : 1-Cyclopentyl-4-{5-[4-(pipéridin-1-ylcarbonyl)phényl]pyridin-2-yl}pipérazine dichlorhydrate

Mode opératoire identique au Stade C de l'Exemple 1 à partir du composé obtenu au Stade B et du composé obtenu dans la Préparation 2.
*Point de fusion : 227-230 °C*
*Microanalyse élémentaire :*

| | *C* | *H* | *N* | *Cl* |
|---|---|---|---|---|
| *% théorique* | *63,54* | *7,38* | *11,40* | *14,43* |
| *% expérimental* | *63,45* | *7,42* | *11,31* | *14,46* |

### Exemple 17 : 1-Isopropyl-4-{5-[4-(pipéridin-1-ylcarbonyl)phényl]pyridin-2-yl}pipérazine dichlorhydrate

Mode opératoire identique au Stade C de l'Exemple 16 en remplaçant le produit obtenu dans la Préparation 2 par le composé obtenu dans la Préparation 1.
*Point de fusion : 240-243 °C*
*Microanalyse élémentaire :*

| | *C* | *H* | *N* | *Cl* |
|---|---|---|---|---|
| *% théorique* | *61,93* | *7,36* | *12,04* | *15,23* |
| *% expérimental* | *62,14* | *7,35* | *11,62* | *15,33* |

### Exemple 18 : 1-Méthyl-4-{5-[4-(pipéridin-1-ylsulfonyl)phényl]pyridin-2-yl}pipérazine dichlorhydrate

Le produit du Stade B de l'Exemple 2 est mis en réaction avec le composé obtenu dans la Préparation 6 selon les conditions décrites au Stade C de l'Exemple 1.
*Point de fusion : 250-255 °C*
*Microanalyse élémentaire :*

| | *C* | *H* | *N* | *S* | *Cl* |
|---|---|---|---|---|---|
| *% théorique* | *53,27* | *6,39* | *11,83* | *6,77* | *14,98* |
| *% expérimental* | *53,51* | *6,40* | *11,82* | *6,71* | *15,16* |

### Exemple 19 : N-Cyclopropyl-4-[6-(4-isopropylpipérazin-1-yl)pyridin-3-yl]benzène sulfonamide dichlorhydrate

### Stade A : N-Cyclopropyl-4-iodobenzènesulfonamide

Mode opératoire identique au Stade A de l'Exemple 1 en remplaçant la morpholine par la cyclopropylamine.

### Stade B : N-Cyclopropyl-4-(4,4,5,5-tétraméthyl-1,3,2-dioxaborolan-2-yl)benzènesulfonamide

Mode opératoire identique au Stade B de l'Exemple 9 à partir du produit obtenu au stade A.
*Point de fusion :* 99 °C

### Stade C : N-Cyclopropyl-4-[6-(4-isopropylpipérazin-1-yl)pyridin-3-yl]benzène sulfonamide dichlorhydrate

Mode opératoire identique au Stade C de l'Exemple 1 à partir du composé obtenu au stade B.
*Point de fusion : 269 °C*
*Microanalyse élémentaire :*

| | *C* | *H* | *N* | *S* | *Cl* |
|---|---|---|---|---|---|
| *% théorique* | *53,27* | *6,39* | *11,83* | *6,77* | *14,98* |
| *% expérimental* | *53,22* | *6,44* | *11,66* | *6,51* | *14,98* |

### Exemple 20 : N-(tert-Butyl)-4-[6-(4-isopropylpipérazin-1-yl)pyridin-3-yl]benzène sulfonamide dichlorhydrate

### Stade A : N-(tert-Butyl)-4-iodobenzènesulfonamide

Mode opératoire identique au Stade A de l'Exemple 1 en remplaçant la morpholine par la *tert*-butylamine.
*Point de fusion : 121 °C*

### Stade B : N-(tert-Butyl)-4-(4,4,5,5-tétraméthyl-1,3,2-dioxaborolan-2-yl)benzènesulfonamide

Mode opératoire identique au Stade B de l'Exemple 9 à partir du produit obtenu au stade A.

### Stade C : N-(tert-Butyl)-4-[6-(4-isopropylpipérazin-1-yl)pyridin-3-yl]benzène sulfonamide dichlorhydrate

Mode opératoire identique au Stade C de l'Exemple 1 à partir du composé obtenu au stade B.
*Point de fusion : 215-230 °C*
*Microanalyse élémentaire :*

| | *C* | *H* | *N* | *S* | *Cl* |
|---|---|---|---|---|---|
| *% théorique* | *53,98* | *7, 00* | *11,45* | *6,55* | *14,48* |
| *% expérimental* | *54,19* | *7,05* | *11,16* | *5,25* | *14,52* |

### Exemple 21 : 4-({4-[6-(4-Isobutylpiperazin-1-yl)pyridin-3-yl]phenyl}sulfonyl) morpholine dichlorhydrate

Le produit obtenu au stade B de l'Exemple 1 est remis en réaction avec le composé obtenu dans la Préparation 7 selon les conditions décrites au Stade C de l'Exemple 1.
*Point de fusion : 137°C*
*Microanalyse élémentaire :*

| | *C* | *H* | *N* | *S* | *Cl* |
|---|---|---|---|---|---|
| *% théorique* | *53, 76* | *6, 65* | *10,90* | *6, 24* | *13,11* |
| *% expérimental* | *54,14* | *6,57* | *10,74* | *6, 06* | *13,04* |

### Exemple 22 : 1-Isopropyl-4-({4-[6-(4-isopropylpiperazin-1-yl)pyridin-3-yl]phenyl}sulfonyl) piperazine, trichlorhydrate

### Stade A : 1-[(4-Iodophényl)sulfonyl]-4-isopropylpipérazine

Mode opératoire identique au Stade A de l'Exemple 1 en remplaçant la morpholine par la 1-isopropylpipérazine.
*Point de fusion : 139 °C*

### Stade B : 1-Isopropyl-4-{[4-(4,4,5,5-tétraméthyl-1,3,2-dioxaborolan-2-yl)phényl]sulfonyl}pipérazine

Mode opératoire identique au Stade B de l'Exemple 9 à partir du produit obtenu au Stade A.

### Stade C : 1-Isopropyl-4-({4-[6-(4-isopropylpipérazin-1-yl)pyridin-3-yl]phényl}sulfonyl) pipérazine, trichlorhydrate

Mode opératoire identique au Stade C de l'Exemple 1 à partir du produit obtenu au Stade B.
*Point de fusion : 290 °C*
*Microanalyse élémentaire :*

| | *C* | *H* | *N* | *S* | *Cl* |
|---|---|---|---|---|---|
| *% théorique* | *51,68* | *6,94* | *12,05* | *5,52* | *18,3* |
| *% expérimental* | *51,35* | *7,39* | *11,77* | *5,35* | *18,5* |

### Exemple 23 : 4-[6-(4-Isopropylpipérazin-1-yl)pyridin-3-yl]benzènesulfonamide dichlorhydrate

### Stade A : 4-Iodobenzènesulfonamide

Mode opératoire identique au Stade A de l'Exemple 1 en remplaçant la morpholine par de l'ammoniac gaz.
*Point de fusion : 173 °C*

### Stade B : 4-(4,4,5,5-Tétraméthyl-1,3,2-dioxaborolan-2-yl)benzène sulfonamide

Mode opératoire identique au Stade B de l'Exemple 9 à partir du produit obtenu au Stade A.

### Stade C : 4-[6-(4-Isopropylpipérazin-1-yl)pyridin-3-yl]benzène sulfonamide dichlorhydrate

Mode opératoire identique au Stade C de l'Exemple 1 à partir du produit obtenu au Stade B.
*Point de fusion : 297-301° C*
*Microanalyse élémentaire :*

| | *C* | *H* | *N* | *S* | *Cl* |
|---|---|---|---|---|---|
| *% théorique* | *49,88* | *6,05* | *12,93* | *7,4* | *16,36* |
| *% expérimental* | *50,05* | *6,21* | *12,58* | *7,39* | *16,46* |

### Exemple 24 : 4-({4-[6-(4-Isopropylpipérazin-1-yl)pyridin-3-yl]phényl}sulfonyl) thiomorpholine 1,1-dioxide dichlorhydrate

A une suspension de 400 mg du produit obtenu dans l'Exemple 10 (0,76 mmol) dans un mélange de 3 ml d'acétone et 12 ml d'eau sont ajoutés 266 mg de 4-méthylmorpholine N-oxide (2,27 mmol) et 34 µl d'une solution de tétroxyde d'osmium 2,5% dans *t*BuOH. Après 16 h d'agitation à température ambiante la réaction est traitée par une solution saturée de bisulfite de sodium et d'hydrogénocarbonate de sodium 10%. Le mélange est extrait par du CH₂Cl₂, les phases organiques séchées sur MgSO₄ et évaporées sous pression réduite. Le résidu est traité au méthanol chlorhydrique pour conduire après filtration au produit du titre sous la forme d'un solide blanc.
*Point de fusion : 278-280 °C*
*Microanalyse élémentaire :*

| | *C* | *H* | *N* | *S* | *Cl* |
|---|---|---|---|---|---|
| *% théorique* | *47,91* | *5,85* | *10,16* | *11,63* | *12,86* |
| *% expérimental* | *48,57* | *5,68* | *10,08* | *11,73* | *13,58* |

### Exemple 25 : 1-Ethyl-4-({4-[6-(4-isopropylpipérazin-1-yl)pyridin-3-yl]phényl}sulfonyl)pipérazine, trichlorhydrate

### Stade A : 1-[(4-Iodophényl)sulfonyl]-4-éthyllpipérazine

Mode opératoire identique au Stade A de l'Exemple 1 en remplaçant la morpholine par la 1-éthylpipérazine.
*Point de fusion : 148 °C*
*Microanalyse élémentaire :*

| | *C* | *H* | *N* | *S* | *I* |
|---|---|---|---|---|---|
| *% théorique* | *37,90* | *4,51* | *7,37* | *8,43* | *33,37* |
| *% expérimental* | *37, 74* | *4,50* | *7,16* | *8,22* | *31,85* |

### Stade B : 1-Ethyl-4-{[4-(4,4,5,5-tétraméthyl-1,3,2-dioxaborolan-2-yl)phényl] sulfonyl}pipérazine

Mode opératoire identique au Stade B de l'Exemple 9 à partir du produit obtenu au Stade A.

### Stade C : 1-Ethyl-4-({4-[6-(4-isopropylpipérazin-1-yl)pyridin-3-yl]phényl} sulfonyl)pipérazine, trichlorhydrate

Mode opératoire identique au Stade C de l'Exemple 1 à partir du produit obtenu au Stade B.
*Point de fusion : 249 °C*
*Microanalyse élémentaire :*

| | *C* | *H* | *N* | *S* | *Cl* |
|---|---|---|---|---|---|
| *% théorique* | *50,84* | *6,75* | *12,35* | *5,66* | *18,76* |
| *% expérimental* | *50,33* | *6,53* | *11,84* | *5,26* | *18,76* |

### Exemple 26 : 4-({4-[6-(4-Isopropylpipérazin-1-yl)pyridin-3yl]phényl}sulfonyl) Thiomorpholine 1-oxide dichlorhydrate

A une solution de 183 ml de NaIO₄ (0,86 mmol) dans 8 ml d'eau sont ajoutés 424 mg du produit de l'Exemple 10 et la réaction est agitée 1 h à température ambiante. Le mélange est extrait par du CH₂Cl₂, les phases organiques sont séchées sur MgSO₄. Après évaporation sous pression réduite le résidu est traité au méthanol chlorhydrique pour conduire après filtration au produit du titre sous la forme d'un solide blanc.
*Point de fusion : 265 °C*
*Microanalyse élémentaire :*

| | *C* | *H* | *N* | *S* | *Cl* |
|---|---|---|---|---|---|
| *% théorique* | *49,68* | *6, 04* | *10,53* | *12,06* | *12,66* |
| *% expérimental* | *49,91* | *6,14* | *10,03* | *11,95* | *12,39* |

### Exemple 27 : 1-{5-[4-(Aziridin-1-ylsulfonyl)phényl]pyridin-2-yl}-4-isopropylpipérazine, dichlorydrate

### Stade A : 1-Cyclopropyl-4-[(4-iodophényl)sulfonyl]pipérazine

Mode opératoire identique au Stade A de l'Exemple 1 en remplaçant la morpholine par la 1-cyclopropylpipérazine.
*Point de fusion : 169 °C*

### Stade B : 1-Cyclopropyl-4-{[4-(4,4,5,5-tétraméthyl-1,3,2-dioxaborolan-2-yl) phényl]sulfonyl}pipérazine

Mode opératoire identique au Stade B de l'Exemple 9 à partir du produit obtenu au Stade A.

### Stade C : 1-Isopropyl-4-({4-[6-(4-isopropylpipérazin-1-yl)pyridin-3-yl]phényl} sulfonyl) pipérazine, trichlorhydrate

Mode opératoire identique au Stade C de l'Exemple 1 à partir du produit obtenu au Stade B.
*Point de fusion : 149 °C*
*Microanalyse élémentaire :*

| | *C* | *H* | *N* | *S* | *Cl* |
|---|---|---|---|---|---|
| *% théorique* | *55,34* | *6,87* | *12,91* | *5,91* | *13,07* |
| *% expérimental* | *55,22* | *7,01* | *12,52* | *5,99* | *12,98* |

### Exemple 28 : 1-Isopropyl-4-(S-{4-[(2-méthylpyrrolidin-1-yl)sulfonyl]phényl}pyridin-2-yl)pipérazine dichlorhydrate

### Stade A : 1-[(4-Iodophényl)sulfonyl]-2-méthylpyrrolidine

Mode opératoire identique au Stade A de l'Exemple 1 en remplaçant la morpholine par la 2-méthylpyrrolidine.
*Point de fusion : 76 °C*

### Stade B: Acide {4-[(2-méthylpyrrolidin-1-yl)sulfonyl]phényl}boronique

Mode opératoire identique au Sstade B de l'Exemple 1 à partir du produit obtenu au Stade A.
*Point de fusion : 125-128°C*

### Stade C : 1-Isopropyl-4-(5-{4-[(2-méthylpyrrolidin-1-yl)sulfonyl]phényl} pyridin-2-yl)pipérazine dichlorhydrate

Mode opératoire identique au Stade C de l'Exemple 1 à partir du produit obtenu au Stade A.
*Point de fusion : 208-213°C*
*Microanalyse élémentaire :*

| | *C* | *H* | *N* | *S* | *Cl* |
|---|---|---|---|---|---|
| *% théorique* | *55,08* | *6,83* | *11,17* | *6,39* | *14,14* |
| *% expérimental* | *55,27* | *6,77* | *10,95* | *6, 27* | *14,47* |

### Exemple 29 : 1-Isopropyl-4-{5-[4-(pipérazin-1-ylsulfonyl)phényl]pyridin-2-yl} pipérazine trichlorhydrate

### Stade A : tert-Butyl 4-[(4-iodophényl)sulfonyl]pipérazine-1-carboxylate

Mode opératoire identique au Stade A de l'Exemple 1 en remplaçant la morpholine par la *tert*-butyl pipérazine-1-carboxylate.

### Stade B : Acide (4-{[4-(tert-butoxycarbonyl)pipérazin-1-yl]sulfonyl}phényl) boronique

Mode opératoire identique au Stade B de l'Exemple 1 à partir du produit obtenu au Stade A.

### Stade C : 4-({4-[6-(4-Isopropylpipérazin-1-yl)pyridin-3-yl]phényl}sulfonyl) pipérazine-1-carboxylate de tert-butyle

Mode opératoire identique au Stade C de l'Exemple 1 à partir du produit obtenu au Stade A.
*Point de fusion : 194 °C*

### Stade D : 1-Isopropyl-4-{5-[4-(pipérazin-1-ylsulfonyl)phényl]pyridin-2-yl} pipérazine trichlorhydrate

La déprotection est réalisée dans un mélange 1/1 de dioxanne et méthanol chlorhydrique.
*Point de fusion : 265-273 °C*
*Microanalyse élémentaire :*

| | *C* | *H* | *N* | *S* | *Cl* |
|---|---|---|---|---|---|
| *% théorique* | *49,03* | *6, 36* | *12,99* | *5,95* | *19,73* |
| *% expérimental* | *49,63* | *6,49* | *12,86* | *6, 2* | *20,39* |

### Exemple 30 : 1-Cyclohexyl-4-({4-[6-(4-isopropylpipérazin-1-yl)pyridin-3-yl]phényl} sulfonyl)pipérazine dichlorhydrate

### Stade A : 1-Cyclohexyl-4-[(4-iodophényl)sulfonyl]pipérazine

Mode opératoire identique au Stade A de l'Exemple 1 en remplaçant la morpholine par la 1-cyclohexylpiperazine.
*Point de fusion: 174-177 °C*
*Microanalyse élémentaire :*

| | *C* | *H* | *N* | *S* | *I* |
|---|---|---|---|---|---|
| *% théorique* | *44,25* | *5,34* | *6,45* | *7,38* | *29,22* |
| *% expérimental* | *44,16* | *5,33* | *6,37* | *7, 00* | *29,07* |

### Stade B : 1-Cyclohexyl-4-{[4-(4,4,5,5-tétraméthyl-1,3,2-dioxaborolan-2-yl) phényl]sulfonyl}pipérazine

Mode opératoire identique au Stade B de l'Exemple 9 à partir du produit obtenu au Stade A.

### Stade C : 1-Cyclohexyl-4-({4-[6-(4-isopropylpipérazin-1-yl)pyridin-3-yl]phényl} sulfonyl)pipérazine dichlorhydrate

Mode opératoire identique au Stade C de l'Exemple 1 à partir du produit obtenu au Stade B.
*Point de fusion : 276-281 °C*
*Microanalyse élémentaire :*

| | *C* | *H* | *N* | *S* | *Cl* |
|---|---|---|---|---|---|
| *% théorique* | *57,52* | *7,41* | *11,98* | *5,48* | *12,13* |
| *% expérimental* | *58,01* | *7,32* | *12,18* | *5,2* | *12,86* |

### Exemple 31 : 1-({4-[6-(4-Isopropylpipérazin-1-yl)pyridin-3-yl]phényl}sulfonyl) pipéridin-4-one dichlorhydrate

### Stade A : 8-[(4-Iodophényl)sulfonyl]-1,4-dioxa-8-azaspiro[4.5]décane

Mode opératoire identique au Stade A de l'Exemple 1 en remplaçant la morpholine par la 1,4-dioxa-8-azaspiro[4.5]décane.
*Point de fusion : 166-169 °C*

### Stade B : [4-(1,4-dioxa-8-azaspiro[4.5]dec-8-ylsulfonyl)phenyl]boronic acid

Mode opératoire identique au Stade B de l'Exemple 1 à partir du produit obtenu au Stade A.
*Point de fusion : 146-148 °C*

### Stade C : 8-({4-[6-(4-Isopropylpipérazin-1-yl)pyridin-3-yl]phényl}sulfonyl)-1,4-dioxa-8-azaspiro[4.5]décane

Mode opératoire identique au Stade C de l'Exemple 1 à partir du produit obtenu au Stade B.
*Point de fusion : 215 °C*
*Microanalyse élémentaire :*

| | *C* | *H* | *N* | *S* |
|---|---|---|---|---|
| *% théorique* | *61,70* | *7,04* | *11,51* | *6,59* |
| *% expérimental* | *61,28* | *7,05* | *11,54* | *6,58* |

### Stade D : 1-({4-[6-(4-Isopropylpipérazin-1-yl)pyridin-3-yl]phényl}sulfonyl) pipéridin-4-one dichlorhydrate

Une suspension de 400 mg du produit obtenu au Stade C (0,82 mmol) dans 5 ml de HCl 1N est agitée 1 h à 80°C. Après neutralisation du milieu réactionnel par NaHCO₃ 10%, le précipité est filtré, rincé par de l'eau et séché. Le solide blanc est mis en suspension dans l'éthanol et est dissous par addition de méthanol chlorhydrique. La solution est évaporée à sec, le résidu est repris dans un mélange éthanol/éther éthylique pour conduire après filtration au produit attendu.
*Point de fusion : >260 °C*
*Microanalyse élémentaire :*

| | *C* | *H* | *N* | *S* | *Cl* |
|---|---|---|---|---|---|
| *% théorique* | *53,59* | *6, 26* | *10,87* | *6, 22* | *13,75* |
| *% expérimental* | *54,25* | *6, 25* | *10,84* | *6,51* | *13,48* |

### Exempte 32 : 1-Isopropyl-4-(5-{4-[(2-méthylpyrrolidin-1-yl)sulfonyl]phényl}pyridin-2-yl)pipérazine dichlorhydrate énantiomère 1

Les 2 énantiomères du composé décrit dans l'Exemple 28 (sous forme de base libre) sont séparés par chromatographie chirale sur colonne Chiralpak AD en utilisant comme solvant d'élution un mélange méthanol/acétonitrile/diéthylamine 150/850/1. Les chlorhydrates sont obtenus par traitement au méthanol chlorhydrique.

### Enantiomère 1 :

*Point de fusion : 243-247 °C*
*Microanalyse élémentaire :*

| | *C* | *H* | *N* | *S* | *Cl* |
|---|---|---|---|---|---|
| *% théorique* | *55,08* | *6,83* | *11,17* | *6,39* | *14,14* |
| *% expérimental* | *55,31* | *6,84* | *10,96* | *6,37* | *14,58* |

### Exemple 33 : 1-Isopropyl-4-(5-{4-[(2-méthylpyrrolidin-1-yl)sulfonyl]phényl}pyridin-2-yl)pipérazine dichlorhydrate énantiomère 2

Les 2 énantiomères du composé décrit dans l'Exemple 28 (sous forme de base libre) sont séparés par chromatographie chirale sur colonne Chiralpak AD en utilisant comme solvant d'élution un mélange méthanol/acétonitrile/diéthylamine 150/850/1. Les chlorhydrates sont obtenus par traitement au méthanol chlorhydrique.

### Enantiomère 2 :

*Point de fusion : 245-249 °C*
*Microanalyse élémentaire :*

| | *C* | *H* | *N* | *S* | *Cl* |
|---|---|---|---|---|---|
| *% théorique* | *55,08* | *6,83* | *11,17* | *6,39* | *14,14* |
| *% expérimental* | *55,41* | *6,75* | *11,12* | *6,32* | *14,85* |

### Exemple 34 : 2-({4-[6-(4-Isopropylpipérazin-1-yl)pyridin-3-yl]phényl}sulfonyl)-5-méthyl-2,5-diazabicyclo[2.2.1]heptane

### Stade A : 2-[(4-Iodophényl)sulfonyl]-5-méthyl-2,5-diazabicyclo[2.2.1]heptane

Mode opératoire identique au Stade A de l'Exemple 1 en remplaçant la morpholine par le 2-méthyl-2,5-diazabicyclo[2.2.1]heptane.
*Point de fusion : 149-152 °C*

### Stade B : 2-Méthyl-5-{[4-(4,4,5,5-tétraméthyl-1,3,2-dioxaborolan-2-yl) phényl] sulfonyl}-2,5-diazabicyclo[2.2.1]heptane

Mode opératoire identique au Stade B de l'Exemple 9 à partir du produit obtenu au Stade A.

### Stade C : 2-({4-[6-(4-Isopropylpipérazin-1-yl)pyridin-3-yl]phényl}sulfonyl)-5-méthyl-2,5-diazabicyclo[2.2.1]heptane

Mode opératoire identique au Stade C de l'Exemple 1 à partir du produit obtenu au Stade B.
*Point de fusion : 194-198 °C*
*Microanalyse élémentaire :*

| | *C* | *H* | *N* | *S* |
|---|---|---|---|---|
| *% théorique* | *63,27* | *7,30* | *15,37* | *7,04* |
| *% expérimental* | *63,09* | *7,36* | *14,73* | *6,76* |

### Exemple 35 : 1-({4-[6r-(4-Isopropylpipérazin-1-yl)pyridin-3-yl]phényl}sulfonyl)-N,N-diméthylpipéridin-4-amine trichlorhydrate

A une suspension de 242 mg du produit obtenu dans l'Exemple 32 (0,54 mmol) dans 2 ml d'éthanol sont ajoutés 89 mg de chlorhydrate de diméthylamine (1,09 mmol), 153 µl de Et₃N (1,09 mmol), 323 µl de Titanium (IV) isopropoxyde (1,08 mmol). Après 16 h d'agitation à température ambiante, 52 mg de NaCNBH₄ (0,82 mmol) sont ajoutés et l'agitation est poursuivie 5 h à température ambiante. La réaction est traitée par addition d'ammoniaque 28% et le mélange est extrait par du CH₂Cl₂. Les phases organiques sont séchées sur MgSO₄ et après évaporation sous pression réduite le résidu est purifié par chromatographie sur colonne de silice en éluant par un mélange 96/4 CH₂Cl₂/ MeOH. Le trichlorhydrate est obtenu par traitement de la base au méthanol chlorhydrique pour conduire après filtration au produit du titre sous la forme d'un solide blanc.
*Point de fusion : 283-286°C*
*Microanalyse élémentaire :*

| | *C* | *H* | *N* | *S* | *Cl* |
|---|---|---|---|---|---|
| *% théorique* | *51,68* | *6,94* | *12,05* | *5,52* | *18,3* |
| *% expérimental* | *51,81* | *7,08* | *12,08* | *4,81* | *17,86* |

### Exemple 36 : 1-Cyclopentyl-4-(5-{4-[(4-méthylpipérazin-1-yl)sulfonyl]phényl} pyridin-2-yl)pipérazine trichlorhydrate

### Stade A : 4-({4-[6-(4-Cyclopentylpipérazin-1-yl)pyridin-3-yl]phényl}sulfonyl) pipérazine-1-carboxylate de tert-butyle

Le produit obtenu au Stade B de l'Exemple 29 est mis en réaction avec le composé obtenu dans la Préparation 2 selon les conditions décrites au Stade C de l'Exemple 1.
*Point de fusion : 235-238°C*
*Microanalyse élémentaire :*

| | *C* | *H* | *N* | *S* |
|---|---|---|---|---|
| *% théorique* | *62,68* | *7,44* | *12,60* | *5,77* |
| *% expérimental* | *62,56* | *7,46* | *12,36* | *5,89* |

### Stade B : 1-Cyclopentyl-4-{5-[4-(pipérazin-1-ylsulfonyl)phényl]pyridin-2-yl} pipérazine

La déprotection est réalisée dans un mélange 1/1 de dioxanne et méthanol chlorhydrique.

La base est régénérée par traitement avec NaHCO₃ 10%.

### Stade C : 1-Cyclopentyl-4-(5-{4-[(4-méthylpiperazin-1-yl)sulfonyl]phényl} pyridin-2-yl)pipérazine trichlorhydrate

Une suspension de 500 mg du produit obtenu au Stade B (1,10 mmol), 270 mg d'acétate de sodium (3,29 mmol), 66 mg de paraformaldéhyde (2,19 mmol) dans 10 ml d'éthanol est agitée une nuit à température ambiante. A la réaction sont alors rajoutés en plusieurs portions 138 mg de NaCNBH₃ (2,19 mmol) et l'agitation est poursuivie 6 h à température ambiante. Le milieu réactionnel est concentré sous pression réduite, le résidu repris dans HCl 1N et le mélange est agité 30 min à température ambiante. Le mélange est ensuite alcalinisé par addition de NaOH 1N et le précipité blanc formé est filtré. Celui-ci est repris en suspension dans de l'éthanol à chaud et après addition d'éther chlorhydrique, une solution est obtenue pour conduire à la cristallisation à température ambiante du produit du titre.
*Point de fusion : 263-267 °C*
*Microanalyse élémentaire :*

| | *C* | *H* | *N* | *S* | *Cl* |
|---|---|---|---|---|---|
| *% théorique* | *51,86* | *6,61* | *12,09* | *5,54* | *18,37* |
| *% expérimental* | *52,25* | *6,68* | *11,96* | *5,33* | *18,94* |

### Exemple 37 : 1-({4-[6-(4-Isopropylpipérazin-1-yl)pyridin-3-yl]phényl}sutfonyl) pipéridin-4-ol dichlorhydrate

A une suspension de 1 g du produit obtenu dans l'Exemple 32 (2,26 mmol) dans 20 ml de méthanol sont ajoutés en plusieurs portions 257 mg de NaBH₄ et la réaction est agitée 2 h à température ambiante. Après addition de 40 ml d'eau, le milieu réactionnel est extrait par du CH₂Cl₂, les phases organiques sont jointes, séchées sur MgSO₄ et évaporés sous pression réduite. Le résidu est repris en suspension dans l'éthanol et filtré. Le solide est mis en solution dans du méthanol chlorhydrique, la solution est évaporée à sec et le résidu trituré dans de l'éther éthylique pour conduire après filtration au produit du titre.
*Point de fusion : 162 °C*
*Microanalyse élémentaire :*

| | *C* | *H* | *N* | *S* | *Cl* |
|---|---|---|---|---|---|
| *% théorique* | *53,38* | *6, 62* | *10,83* | *6, 2* | *13,7* |
| *% expérimental* | *53,64* | *6,79* | *10,88* | *6, 03* | *12,63* |

### Exemple 38 : 1-Isopropyl-4-(5-{3-[(4-méthylpipérazin-1-yl)sulfonyl]phényl}pyridin-2-yl)pipérazine trichlorhydrate

### Stade A : 4-[(3-Bromophényl)sulfonyl]pipérazine-1-carboxylate de tert-butyle

Mode opératoire identique au Stade A de l'Exemple 1 en remplaçant la morpholine par la *tert*-butyl pipérazine-1-carboxylate et le chlorure de 4-iodobenzènesulfonyle par le chlorure de 3-bromobenzènesulfonyle.

### Stade B : Acide (3-{[4-(tert-butoxycarbonyl)pipérazin-1-yl]sulfonyl}phényl) boronique

Mode opératoire identique au stade B de l'exemple 1 à partir du produit obtenu au stade A.
*Point de fusion :* 225 °C

### Stade C : 4-({3-[6-(4-Isopropylpipérazin-1-yl)pyridin-3-yl]phényl}sulfonyl) pipérazine-1-carboxylate de tert-butyle

Mode opératoire identique au Stade C de l'Exemple 1 à partir du produit obtenu au Stade B.

### Stade D : 1-Isopropyl-4-{5-[3-(pipérazin-1-ylsulfonyl)phényl]pyridin-2-yl}pipérazine

Mode opératoire identique au Stade B de l'Exemple 37 à partir du produit obtenu au Stade C.

### Stade E : 1-Isopropyl-4-(5-{3-[(4-méthylpipérazin-1-yl)sulfonyl]phényl}pyridin-2-yl)pipérazine trichlorhydrate

Mode opératoire identique au Stade C de l'Exemple 37 à partir du produit obtenu au Stade D.
*Point de fusion : 168 °C*
*Microanalyse élémentaire :*

| | *C* | *H* | *N* | *S* | *Cl* |
|---|---|---|---|---|---|
| *% théorique* | *49,96* | *6,56* | *12,66* | *5,8* | *19,23* |
| *% expérimental* | *50,07* | *6,14* | *12,55* | *5, 67* | *19,47* |

### Exemple 39 : 1-(5-{4-[(4-Fluoropipéridin-1-yl)sulfonyl]phényl}pyridin-2-yl)-4-isopropylpipérazine dichlorhydrate

### Stade A : 4-Fluoro-1-[(4-iodophényl)sulfonyl]pipéridine

Mode opératoire identique au Stade A de l'Exemple 1 en remplaçant la morpholine par la 4-fluoropipéridine.
*Point de fusion : 130-133 °C*

### Stade B : Acide {4-[(4-fluoropipéridin-1-yl)sulfonyl]phényl}boronique

Mode opératoire identique au Stade B de l'Exemple 1 à partir du produit obtenu au Stade A.

### Stade C : 1-(5-{4-[(4-Fluoropipéridin-1-yl)sulfonyl]phényl}pyridin-2-yl)-4-isopropylpipérazine dichlorhydrate

Mode opératoire identique au Stade C de l'Exemple 1 à partir du produit obtenu au Stade B.
*Point de fusion : 243-247 °C*
*Microanalyse élémentaire :*

| | *C* | *H* | *N* | *S* | *Cl* |
|---|---|---|---|---|---|
| *% théorique* | *53,18* | *6,4* | *10,78* | *6,17* | *13,65* |
| *% expérimental* | *52,91* | *6,4* | *10,6* | *5,79* | *13,46* |

### Exempte 40 : 4-{4-[6-(4-Isopropylpipérazin-1-yl)pyridin-3-yl]benzoyl}morpholine dichlorhydrate

### Stade A : 4-(4-Iodobenzoyl)morpholine

Mode opératoire identique au Stade A de l'Exemple 16 en remplaçant la pipéridine par la morpholine.

### Stade B : Acide [4-(morpholin-4-ylcarbonyl)phényl]boronique

Mode opératoire identique au Stade B de l'Exemple 1 à partir du composé obtenu au Stade A.
*Point de fusion : 116 °C*

### Stade C : 4-{4-[6-(4-Isopropylpipérazin-1-yl)pyridin-3-yl]benzoyl}morpholine dichlorhydrate

Mode opératoire identique au Stade C de l'Exemple 1 à partir du composé obtenu au Stade B.
*Point de fusion : 224 °C*
*Microanalyse élémentaire :*

| | *C* | *H* | *N* | *Cl* |
|---|---|---|---|---|
| *% théorique* | *59,1* | *6,9* | *11,99* | *15,17* |
| *% expérimental x* | *58,68* | *6,91* | *11,6* | *15,05* |

### Exemple 41 : 1-Isopropyl-4-(5-{4-[(4-méthylpipérazin-1-yl)carbonyl]phényl}pyridin-2-yl)pipérazine trichlorhydrate

### Stade A : 1-(4-Iodobenzoyl)-4-méthylpipérazine

Mode opératoire identique au Stade A de l'Exemple 16 en remplaçant la pipéridine par la 1-méthylpipérazine.

### Stade B : 1-Méthyl-4-[4-(4,4,5,5-tétraméthyl-1,3,2-dioxaborolan-2-yl)benzoyl] pipérazine

Mode opératoire identique au Stade B de l'Exemple 9 à partir du produit obtenu au Stade A.
*Point de fusion :* 92 °C

### Stade C : 1-Isopropyl-4-(5-{4-[(4-méthylpipérazin-1-yl)carbonyl]phényl} pyridin-2-yl)pipérazine trichlorhydrate

Mode opératoire identique au Stade C de l'Exemple 1 à partir du composé obtenu au Stade B.
*Point de fusion : 288 °C*
*Microanalyse élémentaire :*

| | *C* | *H* | *N* | *Cl* |
|---|---|---|---|---|
| *% théorique* | *55,76* | *7, 02* | *13,55* | *20,57* |
| *% expérimental* | *55,40* | *7, 02* | *13,08* | *20,13* |

### Exempte 42 : 1-({4-[6-(4-Isopropylpipérazin-1-yl)pyridin-3-yl]phényl}sulfonyl)-N-méthylpipéridin-4-amine, trichlorhydrate

Mode opératoire identique à l'Exemple 35 mais en remplaçant le chlorhydrate de diméthylamine par la méthylamine 2M dans le méthanol.
*Point de fusion : 284-288 °C*
*Microanalyse élémentaire :*

| | *C* | *H* | *N* | *S* | *Cl* |
|---|---|---|---|---|---|
| *% théorique* | *50,84* | *6,75* | *12,35* | *5, 66* | *18,76* |
| *% expérimental* | *50,87* | *6,81* | *12,13* | *5,23* | *18,91* |

### Exemple 43 : (1S,4S)-5-({4-[6-(4-Isopropylpipérazin-1-yl)pyridin-3-yl]phényl} sulfonyl)-2-oxa-5-azabicyclo[2.2.1]heptane dichlorhydrate

### Stade A : (1S,4S)-5-[(4-Iodophényl)sulfonyl]-2-oxa-5-azabicyclo[2.2.1]heptane

Mode opératoire identique au Stade A de l'Exemple 1 en remplaçant la morpholine par le (1*S*,4*S*)-2-oxa-5-azabicyclo[2.2.1]heptane.
*Point de fusion : 146-148 °C*

### Stade B : Acide {4-[(1S,4S)-2-oxa-5-azabicyclo[2.2.1]hept-5-ylsulfonyl] phényl}boronique

Mode opératoire identique au Stade B de l'Exemple 1 à partir du produit obtenu au Stade A.

### Stade C : 1-(5-{4-[(4-Fluoropipéridin-1-yl)sulfonyl]phényl}pyridin-2-yl)-4-isopropylpipérazine dichlorhydrate

Mode opératoire identique au Stade C de l'Exemple 1, à partir du produit obtenu au Stade B.
*Point de fusion : 238-242 °C*
*Microanalyse élémentaire :*

| | *C* | *H* | *N* | *S* | *Cl* |
|---|---|---|---|---|---|
| *% théorique* | *53,59* | *6,26* | *10,87* | *6,22* | *13,75* |
| *% expérimental* | *53,36* | *6,34* | *10,62* | *5,86* | *13,80* |

### Exemple 44 : 1-({4-[6-(4-Isopropylpipérazin-1-yl)pyridin-3-yl]phényl}sulfonyl) pipéridin-4-amine trichlorhydrate

Mode opératoire identique à l'Exemple 35 en remplaçant la diméthylamine par NH₃.
*Point de fusion : 293-294 °C*
*Microanalyse élémentaire :*

| | *C* | *H* | *N* | *S* | *Cl* |
|---|---|---|---|---|---|
| *% théorique* | *49,96* | *6,56* | *12,66* | *5,80* | *19,23* |
| *% expérimental* | *49,68* | *6,84* | *12,34* | *5,76* | *19,18* |

### ETUDE PHARMACOLOGIQUE DES PRODUITS DE L'INVENTION

### EXEMPLE A : Dosages cérébraux de la N^{τ}-Méthylhistamine chez la souris NMRI

Cette étude réalisée selon la méthode de Taylor et Coll. (Biochem. Pharm., 1992, 44, 1261-1267), a pour objectif d'évaluer l'activité *ex vivo* des composés de la présente invention en tant qu'antagonistes des récepteurs histaminergiques centraux de type H₃. Cette activité est révélée par la mesure, après traitement par voie intrapéritonéale des composés sous étude, des taux centraux de N^{τ}- Méthylhistamine, métabolite principal de l'histamine. Une augmentation des concentrations cérébrales de N^{τ}- Méthylhistamine signe une augmentation du turn-over de l'histamine par blocage des récepteurs histaminergiques centraux de type H₃.

Des souris NMRI (18-20g) sont traitées par voie intrapéritonéale ou orale par les composés de la présente invention ou par leur véhicule (20 ml/kg). Une heure après le traitement pharmacologique, les animaux sont sacrifiés, les cerveaux sont prélevés, congelés dans l'azote liquide, pesés et homogénéisés dans HClO₄ 0,1N à 4°C. Les homogénats sont centrifugés (15000g, 17 min, 4°C). Les surnageants sont récupérés et aliquotés. Les aliquotes sont congelés dans l'azote liquide et stockés à -80°C jusqu'à leur analyse. La détermination des taux cérébraux de N^{τ}- Méthylhistamine est réalisée par dosage radio-immunologique (RIA) à l'aide d'un kit de dosage. Les taux tissulaires de N^{τ-}Méthylhistamine sont exprimés en µg/g de cerveau frais. La comparaison des taux cérébraux de N^{τ}-Méthylhistamine entre les animaux traités par le véhicule (témoins) et les animaux traités par les composés de la présente invention est effectuée par une analyse de variance à un facteur suivie si nécessaire par une analyse complémentaire (test de Dunnett).

Les résultats montrent que les composés de la présente invention sont capables, pour des doses comprises entre 1 et 10 mg/kg PO, d'augmenter de 100% les concentrations cérébrales endogènes de N^{τ}-Méthylhistamine.

A titre d'exemple, les composés des Exemples 1, 5 et 9 administrés à 10 mg/kg PO et le composé de l'Exemple 21 administré à 3 mg/kg PO augmentent respectivement les concentrations cérébrales endogènes de N^{τ}-Méthylhistamine de 105 %, 197 %, 121 % et 168 %. Ces résultats indiquent que les composés de la présente invention sont de puissants antagonistes des récepteurs histaminergiques centraux de type H₃.

### EXEMPLE B : Enregistrements de l'électroencephalogramme chez le rat vigile

Des rats Wistar mâles adultes sont implantés de manière chronique par des électrodes placées à la surface du cortex frontal et pariétal. Des enregistrements de l'électroencephalogramme (EEG) cortical sont effectués chez les rats placés dans des cages dans une pièce isolée phoniquement. Les composés et solvant sont administrés de manière randomisée à 10 h les mêmes jours avec un minimum de 3 jours entre chaque administration, permettant d'utiliser chaque rat comme son propre témoin. La puissance absolue des activités lentes delta (1-4 Hz), qui prédominent pendant le sommeil lent et disparaissent pendant l'éveil et le sommeil paradoxal, est moyennée sur des périodes successives de 30 min. Sur 30 min, des valeurs élevées et basses de la puissance des activités lentes delta sont des signes d'éveil et de sommeil, respectivement.
Les résultats montrent que les composés de la présente invention augmentent l'éveil (diminution des ondes delta) pour des doses comprises entre 0,3 et 3 mg/kg IP.
A titre d'exemple, le composé de l'Exemple 1 administré à la dose de 0,3 mg/kg, induit une diminution significative de la puissance des ondes lentes delta pendant 150 minutes, signe d'activation corticale et d'éveil. A la dose de 3 mg/kg, on observe en plus une latence d'apparition du sommeil : le premier épisode de sommeil lent survient 73 ± 5 minutes après l'administration du composé de l'Exemple 1 alors que dans le groupe contrôle le premier épisode de sommeil lent survient à 45 ± 5 minutes.

## Revendications

1. Composés de formule (I) : 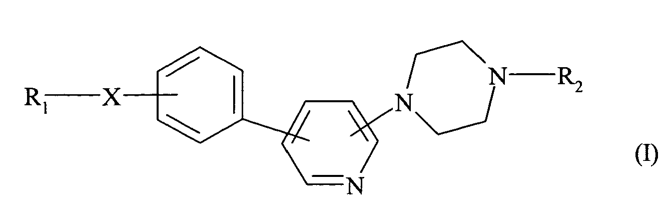 dans laquelle :
X représente un groupement C(O) ou SO₂,
R₁ représente:
- un groupement aryle,
- ou un groupement NR₃R₄ dans lequel R₃ et R₄, identiques ou différents, représentent chacun un atome d'hydrogène ou un groupement alkyle (C₁-C₆) linéaire ou ramifié, cycloalkyle (C₃-C₈) ou cycloalkyl (C₃-C₈) alkyle (C₁-C₆) linéaire ou ramifié,
ou R₃ et R₄ forment ensemble avec l'atome d'azote qui les porte un cycle comportant de 5 à 8 chaînons dont un des atomes de carbone peut être remplacé par un atome d'azote, d'oxygène ou de soufre ou par un groupement SO ou SO₂, le cycle ainsi défini pouvant être ponté par une chaîne alkyle (C₁-C₆) linéaire ou ramifiée, et/ou pouvant être éventuellement substitué par un ou plusieurs groupements, identiques ou différents, choisis parmi halogène, alkyle (C₁-C₆) linéaire ou ramifié, cycloalkyle (C₃-C₈), alkoxy (C₁-C₆) linéaire ou ramifié, polyhalogénoalkyle (C₁-C₆) linéaire ou ramifié, carboxy, hydroxy, cyano, oxo, nitro, amino (éventuellement substitué par un ou plusieurs groupements alkyle (C₁-C₆) linéaire ou ramifié),
R₂ représente un groupement alkyle (C₁-C₆) linéaire ou ramifié, un groupement cycloalkyle (C₃-C₈), ou un groupement cycloalkyle (C₃-C₈) alkyle (C₁-C₆) dans lequel la partie alkyle peut être linéaire ou ramifiée,
étant entendu que :
par groupement aryle, on comprend les groupements phényle, naphtyle et biphényle, ces groupements étant éventuellement substitués par un ou plusieurs groupements, identiques ou différents, choisis parmi halogène, alkyle (C₁-C₆) linéaire ou ramifié, alkoxy (C₁-C₆) linéaire ou ramifié, polyhalogénoalkyle (C₁-C₆) linéaire ou ramifié, carboxy, hydroxy, cyano, nitro, amino (éventuellement substitué par un ou plusieurs groupements alkyle (C₁-C₆) linéaire ou ramifié),
leurs énantiomères et diastéréoisomères ainsi que leurs sels d'addition à un acide ou à une base pharmaceutiquement acceptable.

2. Composés de formule (I), selon la revendication 1, pour lesquels R₃ et R₄ forment ensemble avec l'atome d'azote qui les porte un cycle comportant de 5 à 8 chaînons dont un des atomes de carbone peut être remplacé par un atome d'azote, d'oxygène ou de soufre ou par un groupement SO ou SO₂, le cycle ainsi défini pouvant être ponté par une chaîne alkyle, leurs énantiomères et diastéréoisomères ainsi que leurs sels d'addition à un acide ou à une base pharmaceutiquement acceptable.

3. Composés de formule (I), selon la revendication 1, pour lesquels R₁ représente un groupement morpholinyle, thiomorpholinyle, pipéridinyle, pipérazinyle, 4-(alkyl)pipérazinyle, pyrrolidinyle, 2-(alkyl)-2,5-diazabicyclo[2.2.1]heptanyle, ou 2-oxa-5-azabicyclo[2.2.1]heptanyle, leurs énantiomères et diastéréoisomères ainsi que leurs sels d'addition à un acide ou à une base pharmaceutiquement acceptable.

4. Composés de formule (I), selon la revendication 1, pour lesquels X représente un groupement SO₂, leurs énantiomères et diastéréoisomères ainsi que leurs sels d'addition à un acide ou à une base pharmaceutiquement acceptable.

5. Composés de formule (I), selon la revendication 1, pour lesquels R₂ représente le groupement isopropyle, leurs énantiomères et diastéréoisomères ainsi que leurs sels d'addition à un acide ou à une base pharmaceutiquement acceptable.

6. Composé de formule (I), selon la revendication 1, qui est le 4-({4-[6-(4-isopropyl pipérazin-1-yl)pyridin-3-yl]phényl}sulfonyl)morpholine dichlorhydrate ainsi que ses sels d'addition à un acide ou à une base pharmaceutiquement acceptable.

7. Procédé de préparation des composés de formule (I), selon la revendication 1, **caractérisé en ce que** l'on utilise comme produit de départ le composé de formule (II) : 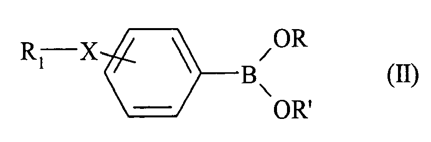 dans laquelle R₁ et X sont tels que définis dans la formule (I) et R et R', identiques ou différents, représentent chacun un atome d'hydrogène ou un groupement alkyle (C₁-C₆) linéaire ou ramifié, ou forment ensemble une chaîne alkylène (C₁-C₆) linéaire ou ramifiée,
sur lequel on condense en présence de palladium (0) un composé de formule (III) : 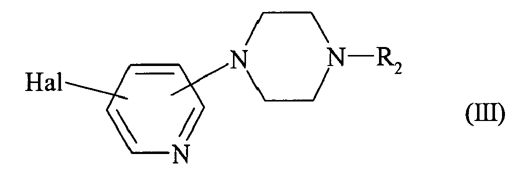 dans laquelle R₂ est tel que défini dans la formule (I) et Hal représente un atome d'halogène,
pour conduire au composé de formule (I),
composé de formule (I) que l'on purifie, le cas échéant, selon une technique classique de purification, dont on sépare éventuellement les isomères, selon une technique classique de séparation, et que l'on transforme, si on le souhaite, en ses sels d'addition, à un acide ou à une base pharmaceutiquement acceptable.

8. Compositions pharmaceutiques contenant comme principe actif un composé selon l'une quelconque des revendications 1 à 6 en combinaison avec un ou plusieurs excipients ou véhicules inertes, non toxiques, pharmaceutiquement acceptables.

9. Compositions pharmaceutiques selon la revendication 8 contenant comme principe actif un composé selon l'une quelconque des revendications 1 à 6 utiles pour la synthèse d'un médicament en tant qu'antagoniste des récepteurs histaminergiques centraux de type H₃.

10. Compositions pharmaceutiques selon la revendication 8 contenant au moins un principe actif selon l'une quelconque des revendications 1 à 6 utiles, en tant que médicament, dans le traitement des déficits cognitifs associés au vieillissement cérébral et aux maladies neurodégénératives, ainsi que dans le traitement des troubles de l'humeur, des crises convulsives, du syndrome d'hyperactivité avec déficits attentionnels, de l'obésité, de la douleur et des états narcoleptiques.

11. Compositions pharmaceutiques selon la revendication 8 contenant au moins un principe actif selon l'une quelconque des revendications 1 à 6 utiles, en tant que médicament, dans le traitement des déficits cognitifs associés à la maladie d'Alzheimer, la maladie de Parkinson, la maladie de Pick, la maladie de Korsakoff, et les démences frontales et sous-corticales d'origines vasculaires ou autres.
